Europäisches Patentamt

(19) European Patent Office

Office européen des brevets

(11) Publication number: **0 010 880**
**B1**

# EUROPEAN PATENT SPECIFICATION

(12)

(45) Date of publication of patent specification: **01.02.84**

(51) Int. Cl.³: **A 61 M 16/00**

(21) Application number: **79302138.7**

(22) Date of filing: **08.10.79**

(54) **Cuffed Endotracheal and Tracheostomy Tubes.**

(30) Priority: **09.10.78 GB 3982078**

(43) Date of publication of application:
**14.05.80 Bulletin 80/10**

(45) Publication of the grant of the patent:
**01.02.84 Bulletin 84/5**

(84) Designated Contracting States:
**DE FR GB IT NL SE**

(56) References cited:
**DE - A - 2 201 867**
**DE - B - 1 566 625**
**US - A - 3 731 692**
**US - A - 3 799 173**
**US - A - 3 854 484**
**US - A - 4 091 816**

(73) Proprietor: **DOVECOURT LIMITED**
**17b Curzon Street**
**London W1 (GB)**

(72) Inventor: **Leigh, Julian Meyer**
**25 Orchard Road Shalford**
**Guildford Surrey, GU4 8ER (GB)**

(74) Representative: **Dixon, Donald Cossar et al,**
**Gee & Co. Chancery House Chancery Lane**
**London WC2A 1QU (GB)**

Courier Press, Leamington Spa, England.

## Cuffed Endotracheal and Tracheostomy Tubes

This invention relates to cuffed endotracheal and tracheostomy tubes but for the sake of brevity the term "endotracheal tube" is used subsquently in this Specification to refer also to tracheostomy tubes where the context so admits.

An endotracheal tube fitted with an inflatable cuff is used to intubate a patient when it is desired to form a seal between the tube and the inner sufface of the tracheal wall. The seal functions to prevent escape of gases from the lungs, thus enabling intermittent positive pressure ventilation (IPPV) to be performed in anaesthesia or intensive care, and to prevent aspiration of fluids into the lungs.

Endotracheal tubes with inflatable cuffs were introduced into anaesthetic practice in 1928 (Guedel & Waters, Curr. Res. Anes, 7, 238) and about thirty years ago pathological changes were noted in tracheal mucosa after as little as two hours (Dwyer et al 1949, Anesthesiology, 10, 714). About ten years ago (Cooper & Grillo 1969, Ann. Surg, 169, 334) attention was focussed on tracheal stenosis as the ultimate pathlogical effect of length intubations: and arising from this high volume low-pressure cuffs (HVLP) were introduced (Carroll et al 1969, Anesthesiology, 31, 275) to replace low volume high-pressure cuffs (LVHP).

LVHP cuffs have not yet disappeared from clinical practice and the HVLP cuffs do not maintain their desirable characteristics when inflated within the trachea beyond the seal-point (SP), unless fitted with an over-pressure safety balloon (McGinnis et al 1971, Anesth. Analg, 50, 558).

The compliance of inflatable structures of which endotracheal tube cuffs are examples is defined as

$$\text{Change in volume} \over \text{Change in pressure} \quad \text{or} \quad {\Delta V \over \Delta P}$$

and depends on both the nature of the wall material and the volume of the structure.

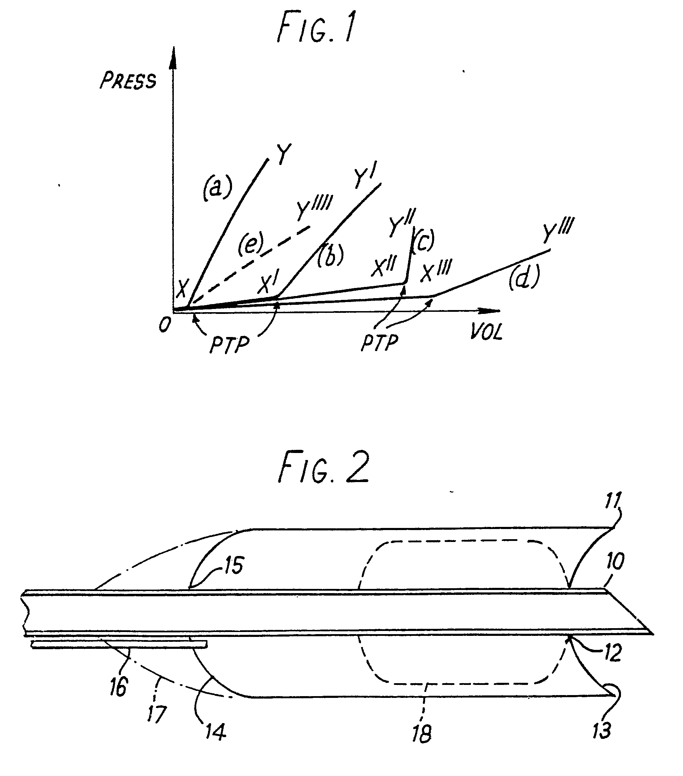

The influence of the elasticity of the wall material and its passive dimensions on the compliance of an inflatable cuff will now be discussed with reference to Figure 1 of the accompanying drawings which shows compliance (V/P) curves for four inflatable cuffs of increasing volume and wall material of different degrees of elasticity as follows:

Cuff a — small volume, inelastic
Cuff b — larger volume, more elastic than a
Cuff c — larger volume than b, less elastic then a
Cuff d — larger volume than c, more elastic then c

Cuff e — equal volume to a, more elastic than a

The V/P relationship of a cuff basically consists of two phases designated on the curves as $o \rightarrow x$ and $x \rightarrow y$. From $o \rightarrow x$ volume increases greatly in relation to pressure as the passive structure of the cuff is inflated until $x$, which is termed the pressure take-off point (PTP), is reached when the elastic properties of the wall come into play. As the passive volume of the cuff increases, the point $x$ (PTP) moves to the right irrespective of the stiffness of the cuff wall material. Therefore if the cuff meets the trachea before $x$ is reached intra-cuff pressure (ICP) and lateral wall pressure (LWP) will be low: if not, the seal-point will be on the steeper portion of the curve and ICP will be high, and LWP tend to be high.

The slope of $xy$ depends upon both the stiffness of the wall and the overall volume of the cuff. If the wall material is very stiff V/P will be small (cuffs a and c): if the wall material is very elastic V/P will be large (cuff d). For a given wall material, V/P is also increased for an increase in volume. Accordingly a cuff with a large passive volume and a very elastic wall will ensure a low ICP and a low LWP at the seal-point. Additionally, as seen, the larger the volume of the cuff, the less steep will be the slope of $xy$ and the effects of over-inflation will be less. It should be noted that the more the cuff is over-inflated the more the volume increments will follow the V/P relationship of the trachea unless the extra charge is allowed to leak to a series compliance, e.g., a Lanz over-pressure safety balloon.

It will have been appreciated that cuff a is an LVHP cuff and cuff b an HVLP cuff. Cuff e would be a hybrid i.e. LVLP. The proponents of HVLP cuffs surmise that:—

(i) The high compliance mitigates the effect of over-inflation.

(ii) "A long cuff has a larger area of application for the diffusion of the pressure" (Magovern et al 1972), J. Thorac. Cardiovasc. Surg, 64, 747).

US Specification No. 3,854,484 (Jackson) discloses a short, liquid-fillable cuff formed of a hydrophobic (polytetrafluoroethylene) wall material that is permeable to air but non-permeable to the liquid at the sealing pressure, thus allowing air to be displaced through the wall when the cuff is filled with the liquid to extend the cuff into sealing engagement with the tracheal wall.

US Specification No. 3,799,173 (Kamen) discloses a cuff which is caused to press outwardly on the tracheal wall by virtue of a resilient sponge-like material with which it is filled, the cuff being collapsed for insertion by the application of suction. When inflated the cuff is elliptical in longitudinal cross-section and thus has little surface area in contact with the tracheal wall.

US Specification No. 3,731,692 (Goodyear) discloses a short inflatable cuff having an essentially rectangular longitudinal cross-section but with rounded corners, such that in use a major portion of the outer surface of the cuff sealingly engages the tracheal wall; the cuff is however shorter than it is wide. The latex rubber or plastisol wall material must be at least 0.13 mm (0.005″) thick. The *Goodyear* cuff is thus a typical example of the HVLP cuffs discussed above.

From a review of the literature including the above-mentioned US Specifications, it appears that there is an implicit assumption that a seal is achieved only as a result of the cuff being pressed onto the tracheal mucosa by the effects of internal inflation. Efforts have been concentrated on the characteristics of this apposition and its prime dependence on the physical properties, e.g. the elasticity and flexibility, of the cuff material in attempts to improve the seal obtained at lower pressures. This relationship is, of course, important as, in the case of all cuffs, ICP is senses by the trachea once circumferential contact is made. The lateral wall pressure (LWP) is very high at the SP with LVHP cuffs; low LWP's occur with HVLP cuffs at the SP, but when HVLP cuffs are over-inflated — which is probably a universal clinical phenomenon — LWP's rise proportionally to ICP, as modified by the increasing effects of tracheal wall compliance and the upper limit imposed by the operation of an over-pressure safety balloon, if fitted.

There is clinical evidence that it would be desirable to reduce ICP even below the values recommended for HVLP cuffs. It has been surmised that in order to prevent tracheal damage, the cuff should exert a pressure no greater than the capillary perfusion pressure of the mucosa. Although mean capillary blood pressure is known to be about 20 mm Hg, the venous end of the capillary has a pressure of approximately only 12 mm Hg. Therefore obstruction of flow will occur if LWP exerted by a cuff on the trachea is greater than this figure. Venous capillary outflow obstruction causes a rise in arteriolar pressure to overcome its effect. The limits of this rise are not known with certainty for all tissues, but it is probable that venous capillary pressure in the tracheal mucosa cannot exceed 40 mm Hg.

The aim of the present invention is to provide a cuff which can be used without the ill effects which may attend the clinical use of a conventional cuff especially over a long period.

The present invention is founded on the realisation that there are important factors which operate during the creation of a seal between the outer surface of the cuff and the mucosal surface other than those which affect the apposition of the two surfaces. A very important attribute of the seal is that it is achieved at the interface between the solid surface of the cuff and mucus, which is a liquid. The seal therefore is a wet seal and not a dry seal and it follows that surface tension or capillarity effects are operative in 'sticking' the cuff to the tracheal wall and also that the viscosity of the mucus is a highly important physical factor in resisting shear forces when gas is attempting to escape from the trachea during IPPV. It further follows that these forces can be used to reduce or replace the effect of ICP in maintaining a seal.

According to the present invention there is provided a cuffed endotracheal tube cuff which has an external diameter in its passive condition, i.e. fully inflated but with the wall material unstretched, not less than 4.0 times the internal diameter of the tube, and in which the wall material has a wettable external surface with respect to mucous liquids. Such a cuff enables viscosity and surface tension effects to be utilised to achieve, in use, a seal with the tracheal wall at an LWP less than the venous capillary pressure of the mucosa and an ICP equal to or less than the airway pressure during IPPV. To utilise the above-mentioned effects as fully as possible, a cuff according to the present invention has a cylindrical length as much as 8 to 10 times the internal diameter of the tube. In terms of tracheal dimensions the cuff should have a passive diameter 2.0 to 2.5 times that of the trachea and a length up to 80% that of the trachea.

Preferably the surface of the cuff is coated with a liquid having a viscosity, and therefore shear-resistance, greater than that of mucus to minimise the ICP at which the cuff may be operated.

The tendency of the cuff to shear from the tracheal mucosa is influenced not only by the viscosity of the liquid medium at the interface but also by the area of the interface and by the time that the shear forces operate. The large surface area of a cuff according to the invention provides a larger interfacial area which enables the viscosity effects to be fully utilised to assist in achieving a seal. The time that the shear forces operate is of course inherent in the intermittent nature of ventilation and cannot be affected by the design of the cuff.

In order that surface tension forces between liquid molecules of the mucosa and liquid molecules on the cuff should be utilised in augmenting the seal the cuff and the mucosa should be closely opposed.

Moreover the weight to surface area ratio of the cuff wall material should be as low as possible; the lower this ratio, which implies as thin a wall as possible, the easier it will be for surface tension forces to 'stick' the cuff to the mucosa. Another advantage of a very thin wall is that any folds in the cuff will be directed inwardly without creating in the tracheal aspect of the cuff wall ridges which could assist air to leak past the cuff and/or damage the trachea.

A further consequence of the thin wall of a cuff according to the present invention is that airway pressure is transmitted at the carinal end to cause ICP to cycle according to lung compliance; however the cycle is damped because the laryngeal aspect of the cuff is exposed to atmospheric pressure and bulges correspondingly. Thus ICP is raised at a time when airway pressure is tending to break the seal between the cuff and the trachea and this increase in ICP counteracts this tendency.

Conventional cuffs are sausage-shaped and a cuff according to the invention may have this form. However a greater resistance to shearing from the tracheal wall is obtained if the carinal end of the cuff is concave.

Although cuffs according to the present invention extend further in the laryngeal direction than do conventional cuffs, preferably for up to 80% of the length of the trachea as mentioned above, they do not normally extend into the sub-glottic region. If however such extension is desirable the laryngeal end is preferably tapered to reduce the risk of trauma.

Although the invention may be put into effect in a variety of ways, two particular embodiments thereof will now be described, by way of example, with reference to Figure 2 of the accompanying drawing which is a diagrammatic axial section through part of an endotracheal tube fitted with a cuff according to the present invention, the cuff being shown in a passive condition.

As shown in Figure 2 the distal end portion of a conventional No 9 endotracheal tube 10 having an internal diameter of 9 mm and a wall thickness of 2.5 mm is fitted with an inflatable cuff 11 having a cylindrical portion with a diameter in the passive condition of 4 cm. The distal junction 12 of the cuff 11 with the tube 10 is inset from the end thereof by the conventional distance of 1 cm and the carinal end of the cuff 11 is formed with a concave end face 13. At its other, laryngeal end the cuff 11 is formed with a convex end face 14 which makes a proximal junction 15 with the tube 10 at a distance of 9 cm from the junction 12. The cuff 11 is inflatable through a tube 16 in the conventional way. The wall material (not more than 0.08 mm thick) of the cuff 11 is that used for family planning devices to BS3704 and its volume in the passive condition about 80 ml. The dimensions given for the cuff 11 relate to a cuff according to the embodiment; for other sizes of tube the dimensions will vary correspondingly.

In an alternative embodiment, the junction 15 is displaced to the left as shown in the drawing so that the cuff 11 extends into the sub-glottic region. To mitigate any possible traumatic effects the cuff 11 is then formed with a tapered extremity as indicated by the chain-dotted lines 17.

The cuff 11 may be coated during manufacture with a wetting agent such as that used to hold contact lenses in position.

For comparison the outline of a typical conventional cuff in the passive condition is shown by the dashed lines 18.

For use the cuff 11, if not coated during manufacture, is coated with a wetting agent, if so desired, and the patient intubated in the conventional manner. The cuff 11 is subsequently inflated by a syringe applied to the end of the tube 16 only enough to cause the cylindrical portion of the cuff 11 to stick to the inner surface of the tracheal wall.

As the internal diameter of the trachea is only 1.5 to 2.0 cm it will be appreciated that the cuff 11 forms a seal with the tracheal mucosa well before its passive diameter is reached. Once effected the seal is maintained, even if ICP is reduced to airway pressure, by surface tension effects between the cuff wall and the tracheal mucosa. By virtue of the low or zero ICP any risk of damage or discomfort to the patient is therefore removed.

It will be appreciated that the above-described cuffs have the high compliance characteristics of cuff *d* in Figure 1. Because of their large passive dimensions compared with those for known cuffs (e.g. a maximum volume for an HVLP cuff fitted to a No 9 tube of 23.73 ml and for a corresponding LVHP cuff of 0.376 ml), circumferential contact is achieved, and a seal formed, with the trachea long before PTP is reached: in other words SP lies on *ox'''*. This being so the cuff wall material may be chosen for its flexibility, lightness and wettability, rather than its elasticity. However, elasticity is important in overcoming the effect of over-inflation beyond the SP. A further consequence of the compliance characteristics of the above-described cuffs is that the need to provide an expensive over-pressure balloon and valve is completely dispensed with.

## Claims

1. A cuffed inflatable endotracheal or tracheostomy tube comprising a tube (10) and a cuff (11) which is fitted therearound and has a generally cylindrical body formed of a flexible wall material with an aperture at each end to allow passage of the tube, the cuff (11) being inflatable by the introduction of gas thereinto characterised in that the cuff (11) has an external diameter in its passive condition, i.e. fully inflated but with the wall material unstretched, not less than 4 times the internal diameter of the tube (10), and that the wall material has a wettable external surface with respect to mucous liquids.

2. A tube as claimed in claim 1, in which the cylindrical length of the cuff (11) is from 8 to 10 times the internal diameter of the tube (10).

3. A tube as claimed in claim 1 or 2, in which the external surface of the wall material has a wetting agent applied thereto.

4. A tube as claimed in claim 1, 2 or 3, in which the wall material has a thickness of not more than 0.08 mm.

## Revendications

1. Tube à manchon endotrachéen ou trachéotomique gonflable comprenant un tube (10) et un manchon (11) monté autour de celui-ci et ayant un corps généralement cylindrique formé en un matériau de paroi flexible avec une ouverture à chaque extrémité pour permettre le passage du tube, le manchon (11) étant gonflable par introduction d'un gaz, caractérisé par le fait que le manchon (11) a un diamètre extérieur en condition passive, c.-à-dire, entièrement gonflé mais sans que la paroi soit détendue, qui n'est pas inférieur à 4 fois le diamètre interne du tube (10), et par le fait que le matériau de paroi a une surface extérieure mouillable par des liquides mucosiques.

2. Tube selon revendication 1, dans lequel la longueur du cylindre de manchon (11) est de 8 a 10 fois le diamètre interne du tube (10).

3. Tube selon revendication 1 ou 2, dans lequel la surface extérieure du matériau de paroi est recouverte d'un mouillant.

4. Tube selon revendication 1, 2 ou 3, dans lequel le matériau de paroi a une épaisseur de pas plus de 0,08 mm.

## Patentansprüche

1. Aufblasbarer Endotracheal- oder Tracheotomy-Tubus mit Ballon, bestehend aus einem Rohr (10) und einem das Rohr umgebenden Ballon (11), der insgesamt die Form eines Zylinders aus biegsamen Wandmaterial hat und eine Öffnung an beiden Enden für den Durchdritt des Rohres aufweist, wobei der Ballon (11) durch Einleiten von Gas aufblasbar ist, dadurch gekennzeichnet, daß der Ballon (11) in passivem Zustand, d.h. vollständig aufgeblasen, aber mit nicht gedehntem Wandmaterial, einen Aufßendurchmesser hat, der mindestens gleich dem 4-fachen des Innendurchmessers des Rohres (10) ist, und daß das Wandmaterial eine durch schleimige Flüssigkeit benetzbare Außenseite besitzt.

2. Tubus nach Anspruch 1, dadurch gekennzeichnet, daß die Zylinderlänge des Ballons (11) dem 8- bis 10 fachen Innendurchmesser des Rohres (10) entspricht.

3. Tubus nach Anspruch 1 oder 2, dadurch gekennzeichnet, daß auf die Außenseite des Wandmaterials ein Netzmittel aufgebracht ist.

4. Tubus nach Anspruch 1, 2 oder 3, dadurch gekennzeichnet, daß das Wandmaterial höchstens 0,08 mm stark ist.

## FIG. 1

## FIG. 2